(19) Europäisches Patentamt
European Patent Office
Office européen des brevets

(11) **EP 4 645 236 A1**

(12) **EUROPEAN PATENT APPLICATION**
published in accordance with Art. 153(4) EPC

(43) Date of publication:
**05.11.2025 Bulletin 2025/45**

(21) Application number: **23909096.2**

(22) Date of filing: **30.06.2023**

(51) International Patent Classification (IPC):
***G06T 17/00*** (2006.01)

(52) Cooperative Patent Classification (CPC):
**A61B 6/00; A61B 6/03; A61B 6/40; A61B 6/42;
G06F 17/18; G06T 5/20; G06T 7/73; G06T 17/00;
G16H 30/00**

(86) International application number:
**PCT/CN2023/105441**

(87) International publication number:
**WO 2024/139152 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.12.2022 CN 202211739643**

(71) Applicant: **Nanovision Technology (Beijing) Co.,
Ltd.
Beijing 100094 (CN)**

(72) Inventors:
• **LUO, Shouhua
  Beijing 100094 (CN)**
• **LI, Yunxiang
  Beijing 100094 (CN)**

(74) Representative: **Wang, Bo
Panovision IP
Ebersberger Straße 3
85570 Markt Schwaben (DE)**

(54) **WEIGHTED ANALYTIC FILTERED BACK PROJECTION RECONSTRUCTION METHOD AND SYSTEM FOR ASYMMETRIC CONE ANGLE ARTIFACTS**

(57) Disclosed in the present invention are a weighted analytic filtered back projection reconstruction method and system for asymmetric cone angle artifacts. The method comprises the following steps: dividing a reconstruction area into a plurality of weight regions on the basis of relative positions of a ray source ring and a detector ring, wherein the ray source ring and the detector ring are mutually staggered to form asymmetric cone angle artifacts; acquiring the projection data volume of voxel points in each weight area irradiated by X-rays; according to the projection data volume of the voxel points in each weight area irradiated by the X-rays, assigning a different initial weight to each weight area; performing smooth transition on the initial weight of each weight area by means of a transition weight to form a final weight assigned to each weight area; and according to different final weights of the weight regions, performing final weighted analytic reconstruction on projection data $p(\alpha, \beta, \gamma)$ collected under large cone beam opening angle geometry, to acquire a back projection image. The method can effectively estimate and compensate for asymmetric cone angle artifacts caused by staggering of a ray source and a detector.

S1: Divide a reconstruction region into a plurality of weight regions on the basis of relative positions of a ray source ring and a detector ring

S2: Acquire the projection data volume of voxel points in each weight region irradiated by X-rays

S3: According to the projection data volume of the voxel points in each weight region irradiated by the X-rays, assign a different initial weight to each weight region

S4: Perform smooth transition on the initial weight of each weight region by means of a transition weight, to form a different final weight assigned to each weight region

S5: According to the different final weights of the weight regions, perform final weighted analytic reconstruction on projection data $p(\alpha, \beta, \gamma)$ collected under large cone beam opening angle geometry, to acquire a back projection image

FIG. 3

**Description**

**BACKGROUND**

**Technical Field**

The present disclosure relates to a weighted analytic filtered back projection reconstruction method for asymmetric cone angle artifacts, also relates to a corresponding back projection reconstruction system, and belongs to the field of medical imaging technologies.

**Related Art**

With the increasing number of detector rows in CT equipment, a problem of cone angle artifacts faced by 3D cone beam reconstruction algorithms has become more prominent. With the emergence of multi-source static CT, due to its unique geometric structure (the center of a ray source ring and the center of a detector ring are not in the same plane), the originally symmetrical cone angles are no longer symmetrical, which brings new challenges to the reconstruction algorithms.

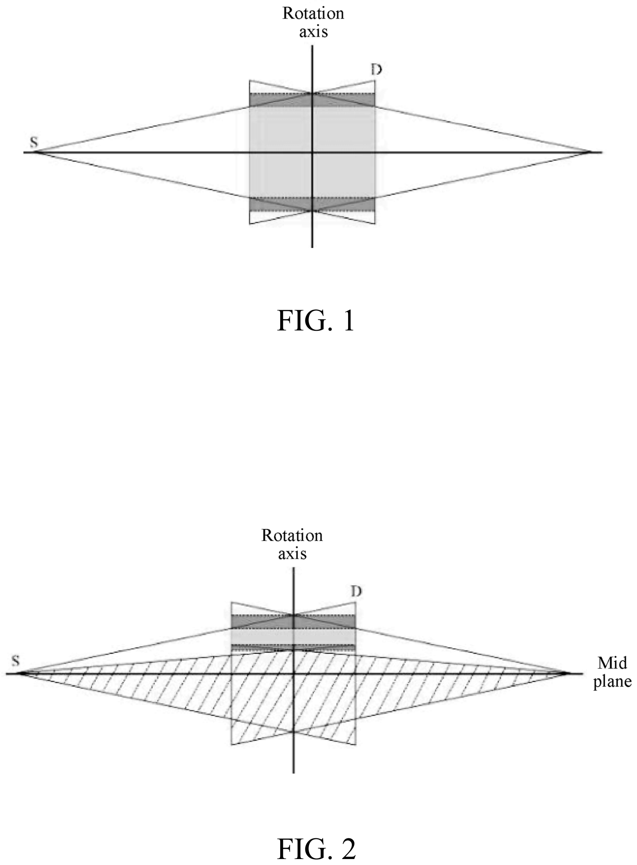

To improve the dose utilization rate of multi-row CT, Grimmer et al. proposed an extended FDK (xFDK) method. The method performs compensation by analytic calculation of the weight of a cone angle region, as shown in FIG. 1. However, the method is only applicable to a case of symmetric cone angles in traditional multi-row spiral CT, and a weight calculation formula is only applicable to a far end cone angle in a case of asymmetric cone angles. When the weight of a near end cone angle is calculated, an incorrect result is obtained, and artifacts cannot be removed.

**SUMMARY**

The most important technical problem to be solved by the present disclosure is to provide a weighted analytic filtered back projection reconstruction method for asymmetric cone angle artifacts.

Another technical problem to be solved by the present disclosure is to provide a back projection reconstruction system for asymmetric cone angle artifacts.

To achieve the above technical objectives, the present disclosure uses the following technical solutions:

According to a first aspect of embodiments of the present disclosure, a weighted analytic filtered back projection reconstruction method for asymmetric cone angle artifacts is provided, including the following steps:

dividing a reconstruction region into a plurality of weight regions on the basis of relative positions of a ray source ring and a detector ring, where the ray source ring and the detector ring are mutually staggered to form asymmetric cone angle artifacts;

acquiring the projection data volume of voxel points in each weight region irradiated by X-rays;

according to the projection data volume of the voxel points in each weight region irradiated by the X-rays, assigning a different initial weight to each weight region;

performing smooth transition on the initial weight of each weight region by means of a transition weight, to form a final weight assigned to each weight region; and

according to the different final weights of the weight regions, performing final weighted analytic reconstruction on projection data $p(\alpha, \beta, \gamma)$ collected under large cone beam opening angle geometry, to acquire a back projection image.

Preferably, the dividing a reconstruction region into a plurality of weight regions on the basis of relative positions of a ray source ring and a detector ring specifically includes:

constructing a space coordinate system by using a center of the detector ring as a circle center O, using an axis of the detector ring as a Z axis, using a horizontal radial direction of the detector ring as an X axis, and using a vertical radial direction of the detector ring as a Y axis,

where in the space coordinate system, cone beam opening angles of the X-rays received by the detector ring fall within a range $[\gamma_2, \gamma_1]$, and a rotation angle $\varphi$ corresponding to coordinates $v = (x, y, z)$ of a voxel point satisfies $x = -r\sin\varphi$ and $y = r\cos\varphi$; and

dividing the reconstruction region into a total of eight weight regions A to H by using the cone beam opening angles of the X-rays on two sides of the Z axis,

where a range $\omega(v, \theta)$ of angles at which the eight weight regions are irradiated by the X-rays is as follows:

EP 4 645 236 A1

$$\omega(\mathbf{v},\theta) = \begin{cases} \phi \ (\text{empty set}) & \text{if } r < c_1 z - R_S \ (\text{Region A}) \\[4pt] \left[ \varphi - \dfrac{\pi}{2} - \theta_1, \varphi + \dfrac{\pi}{2} + \theta_1 \right] & \text{else if } r \ge c_1 z - R_S \ge 0 \ (\text{Region B}) \\[4pt] \left[ \varphi - \dfrac{\pi}{2} - \theta_1, \varphi + \dfrac{\pi}{2} + \theta_1 \right] & \text{else if } c_2 z - R_S \ge r \ge R_S - c_1 z > 0 \ (\text{Region C}) \\[4pt] \left[ \varphi - \pi, \varphi + \pi \right] & \text{else if } r < R_S - c_1 z \text{ and } r \le c_2 z - R_S \ (\text{Region D}) \\[4pt] \left[ \varphi - \dfrac{\pi}{2} - \theta_1, \varphi - \dfrac{\pi}{2} - \theta_2 \right] \cup \left[ \varphi + \dfrac{\pi}{2} + \theta_2, \varphi + \dfrac{\pi}{2} + \theta_1 \right] & \text{else if } r \ge R_S - c_1 z > 0 \text{ and } r > c_2 z - R_S > 0 \ (\text{Region E}) \\[4pt] \left[ \varphi - \pi, \varphi - \dfrac{\pi}{2} - \theta_2 \right] \cup \left[ \varphi + \dfrac{\pi}{2} + \theta_2, \varphi + \pi \right] & \text{else if } R_S - c_1 z > r > c_2 z - R_S > 0 \ (\text{Region F}) \\[4pt] \left[ \varphi - \pi, \varphi - \dfrac{\pi}{2} - \theta_2 \right] \cup \left[ \varphi + \dfrac{\pi}{2} + \theta_2, \varphi + \pi \right] & \text{else if } r \ge c_2 z - R_S \ge 0 \ (\text{Region G}) \\[4pt] \phi \ (\text{empty set}) & \text{else } (\text{Region H}) \end{cases}$$

where $c_1 = \cot \gamma_1$, $c_2 = \cot \gamma_2$, and the definitions of $\theta_1$ and $\theta_2$ are as follows:

$$\theta_1 = \begin{cases} \arcsin \dfrac{R_S - c_1 z}{r} - \arctan \dfrac{\sqrt{r^2 - (R_S - c_1 z)^2}}{c_1 z} & \text{if } r > |R_S - c_1 z| \\[10pt] \operatorname{sgn}(R_S - c_1 z)\dfrac{\pi}{2} & \text{else} \end{cases}$$

$$\theta_2 = \begin{cases} \arcsin \dfrac{R_S - c_2 z}{r} - \arctan \dfrac{\sqrt{r^2 - (R_S - c_2 z)^2}}{c_2 z} & \text{if } r > |R_S - c_2 z| \\[10pt] \operatorname{sgn}(R_S - c_2 z)\dfrac{\pi}{2} & \text{else} \end{cases}$$

where $R_S$ represents a vertical distance from a ray source to a center of an FOV; z represents a Z-axis coordinate of a voxel point v; r represents the length of a vector (x, y) in an XY plane; and $\varphi$ represents an angle formed between the vector (x, y) in the XY plane and the Y axis.

Preferably, the projection data volume $\phi(v, \theta)$ of the voxel points in each weight region irradiated by the X-rays is calculated by the following formula:

$$\Phi(\mathbf{v},\theta) = \begin{cases} 0 & \text{if } r < c_1 z - R_S \text{ (Region A)} \\ \pi + 2\theta_1 & \text{else if } r \geq c_1 z - R_S \geq 0 \text{ (Region B)} \\ \pi + 2\theta_1 & \text{else if } c_2 z - R_S \geq r \geq R_S - c_1 z > 0 \text{ (Region C)} \\ 2\pi & \text{else if } r < R_S - c_1 z \text{ and } r \leq c_2 z - R_S \text{ (Region D)} \\ 2\theta_1 - 2\theta_2 & \text{else if } r \geq R_S - c_1 z > 0 \text{ and } r > c_2 z - R_S > 0 \text{ (Region E)} \\ \pi - 2\theta_2 & \text{else if } R_S - c_1 z > r > c_2 z - R_S > 0 \text{ (Region F)} \\ \pi - 2\theta_2 & \text{else if } r \geq R_S - c_2 z \geq 0 \text{ (Region G)} \\ 0 & \text{else (Region H)} \end{cases}$$

.

Preferably, the assigning a different initial weight to each weight region specifically includes:

according to the projection data volume $\phi(v, \theta)$ of the voxel points in each weight region irradiated by the X-rays, dividing the eight weight regions A to H into: a non-irradiated region, a partially irradiated region, a fully irradiated region, and a non-fixed irradiated region;
assigning an initial weight 0 to the non-irradiated region;
assigning an initial weight $0 < W_{PS}(v, \theta) < 1$ to the partially irradiated region and the non-fixed irradiated region; and
assigning an initial weight $W_{FS}(v, \theta) = 1$ to the fully irradiated region.

Preferably, the weight region A and the weight region H are divided as non-irradiated regions; the weight region B and the weight region G are divided as regions irradiated at an angle less than 180°; the weight region C and the weight region F are divided as regions irradiated at an angle equal to or greater than 180° and less than 360°; the weight region D is divided as a 360° fully irradiated region; and the weight region E is divided as a non-fixed irradiated region.

If the region irradiated at an angle equal to or greater than 180° and less than 360° is the weight region C, i.e., $c_2 z - R_S > R_S - c_1 z$ is satisfied, then

$$W_{PS}(\mathbf{v},\theta) = \begin{cases} 0 & \text{if } \theta < \varphi - \pi/2 - \theta_1 \\ 1 + s(\dfrac{\theta - \varphi + \pi/2}{\theta_1}) & \text{else if } \theta < \varphi - \pi/2 + \theta_1 \\ 2 & \text{else if } \theta < \varphi + \pi/2 - \theta_1 \\ 1 - s(\dfrac{\theta - \varphi - \pi/2}{\theta_1}) & \text{else if } \theta < \varphi + \pi/2 + \theta_1 \\ 0 & \text{else} \end{cases}$$

If the region irradiated at an angle equal to or greater than 180° and less than 360° is the weight region F, i.e., $R_S - c_1 z \geq c_2 z - R_S$ is satisfied, then

$$W_{PS}(\mathbf{v},\theta) = \begin{cases} 2 & \text{if } \theta < \varphi - \pi/2 + \theta_2 \\ 1 + s(\dfrac{\theta - \varphi + \pi/2}{\theta_2}) & \text{else if } \theta < \varphi - \pi/2 - \theta_2 \\ 0 & \text{else if } \theta < \varphi + \pi/2 + \theta_2 \\ 1 - s(\dfrac{\theta - \varphi - \pi/2}{\theta_2}) & \text{else if } \theta < \varphi + \pi/2 - \theta_2 \\ 2 & \text{else} \end{cases}$$

where a function s(t)=sin(πt/2).

For the region irradiated at an angle less than 180° and/or the non-fixed irradiated region,

$$W_{PS}(\mathbf{v},\theta) = \begin{cases} \min(2, \dfrac{2\pi}{\Phi(\mathbf{v},\theta)}) & \text{if } \theta \in \omega(\mathbf{v},\theta) \\ 0 & \text{else} \end{cases}$$

For the 360° fully irradiated region, the same weight $W_{FS}(v, \theta) = 1$ is assigned to all data.

Preferably, the performing smooth transition on the initial weight of each weight region by means of a transition weight, to form a final weight assigned to each weight region specifically includes:

introducing the transition weight $W_T(v, \theta)$ to perform smooth transition on the initial weight of each weight region, to form the final weight $W_C(v,\theta) = (1- W_T(v,\theta))W_{FS}(v,\theta) + W_T(v,\theta)W_{PS}(v,\theta)$,
where for a non-360° fully irradiated region, $W_T(v, \theta)=1$; and
for the 360° fully irradiated region,

$$W_T(\mathbf{v},\theta) = \frac{1}{2}\begin{cases} 0 & \text{if } r < r_0 - \Delta r \\ 1 + s(\dfrac{r - r_0}{\Delta r}) & \text{else if } r \le r_0 + \Delta r \\ 2 & \text{else} \end{cases}$$

where $\Delta r = R_M^2/2R_S$, $r_0 = \min(R_S - c_1 z, c_2 z - R_S) - \Delta r$, and $R_M$ is the radius of the FOV.

Preferably, final weighted analytic reconstruction is performed by the following formula:

$$f(\mathbf{v}) = \frac{1}{2}\int W_C(\mathbf{v},\theta)h(\xi) * p(\theta,\xi,\gamma)\mathrm{d}\theta$$

where $p(\theta, \xi, \gamma)$ is the parallel beam geometric projection data obtained after weighted rearrangement of the $p(\alpha, \beta, \gamma)$, $\xi$ is a projection image horizontal index variable after the rearrangement, and $h(\xi)$ is a filter kernel.

According to a second aspect of the embodiments of the present disclosure, a back projection reconstruction system for asymmetric cone angle artifacts is provided, including a processor and a memory. The processor reads a computer program in the memory, and performs the following operations:

dividing a reconstruction region into a plurality of weight regions on the basis of relative positions of a ray source ring and a detector ring, where the ray source ring and the detector ring are mutually staggered to form asymmetric cone angle artifacts;

according to the projection data volume of the voxel points in each weight region irradiated by the X-rays, assigning a different initial weight to each weight region;

performing smooth transition on the initial weight of each weight region by means of a transition weight, to form a final weight assigned to each weight region; and

according to the different final weights of the weight regions, performing final weighted analytic reconstruction on projection data $p(\alpha, \beta, \gamma)$ collected under large cone beam opening angle geometry, to acquire a back projection image.

Preferably, the ray source ring is formed by a plurality of X-ray sources surrounding in a circle, the detector ring is formed by a plurality of X-ray detectors surrounding in a circle, and the detector ring is arranged on an inner side of the ray source ring.

According to a third aspect of the embodiments of the present disclosure, a computer-readable storage medium including a program instruction is provided. The program instruction, when executed by a processor, implements the weighted analytic filtered back projection reconstruction method as described above.

Compared with the related art, the weighted analytic filtered back projection reconstruction method provided by the present disclosure can effectively estimate and compensate for asymmetric cone angle artifacts caused by staggering of ray sources and detectors. After a reconstruction region is weighted according to the method, a reconstructable range of a single axial scan in static CT can be greatly improved, so that the uniformity and accuracy of a CT value at an axial scan analytic FBP reconstruction edge layer can be significantly improved. The utilization rate of a ray dose in static CT is indirectly improved by expanding the reconstructable range, and the ray dose required for static CT imaging is effectively reduced. In addition, a weighting formula only applicable to a symmetric cone angle configuration system is extended to be also applicable to an asymmetric cone angle configuration system, so that the method can be applied to geometric configuration in static CT.

## BRIEF DESCRIPTION OF THE DRAWINGS

FIG. 1 is a schematic diagram of a geometric configuration to which an xFDK weighting method is applicable in the related art;

FIG. 2 is a schematic diagram of a geometric configuration to which a bixFDK weighting method is applicable according to an embodiment of the present disclosure;

FIG. 3 is a flowchart of a weighted analytic filtered back projection reconstruction method for asymmetric cone angle artifacts provided in an embodiment of the present disclosure;

FIG. 4 is a slice view of a ray source ring and a detector ring in a space coordinate system according to an embodiment of the present disclosure;

FIG. 5 is a sagittal view of a ray source ring and a detector ring in a space coordinate system according to an embodiment of the present disclosure;

FIG. 6 is a schematic diagram of regions obtained by dividing a reconstruction region according to an embodiment of the present disclosure;

FIG. 7 is a schematic diagram of a projection image reconstructed by a generic FDK algorithm;

FIG. 8 is a schematic diagram of a projection image reconstructed by a generic xFDK weighting algorithm;

FIG. 9 is a schematic diagram of a projection image reconstructed by a bixFDK weighting algorithm according to an embodiment of the present disclosure; and

FIG. 10 is schematic structural diagram of a back projection reconstruction system for asymmetric cone angle artifacts provided in an embodiment of the present disclosure.

## DETAILED DESCRIPTION

The technical contents of the present disclosure will be specifically described in detail below with reference to the accompanying drawings and specific embodiments.

As shown in FIG. 2, an embodiment of the present disclosure first provides a weighted analytic filtered back projection reconstruction method for asymmetric cone angle artifacts, which compensate for a cone angle shadow caused by incomplete data based on an analytic derivation back projection weighting factor calculation method, to separately process a near-end cone angle and a far-end cone angle of the asymmetric cone angle artifacts, so that both ends can obtain correct weights, and thereby the cone angle is correctly compensated for. Compared with an existing cone angle weighted reconstruction algorithm, the method has advantages of accurate compensation, higher calculation speed, and introduction of no other artifacts.

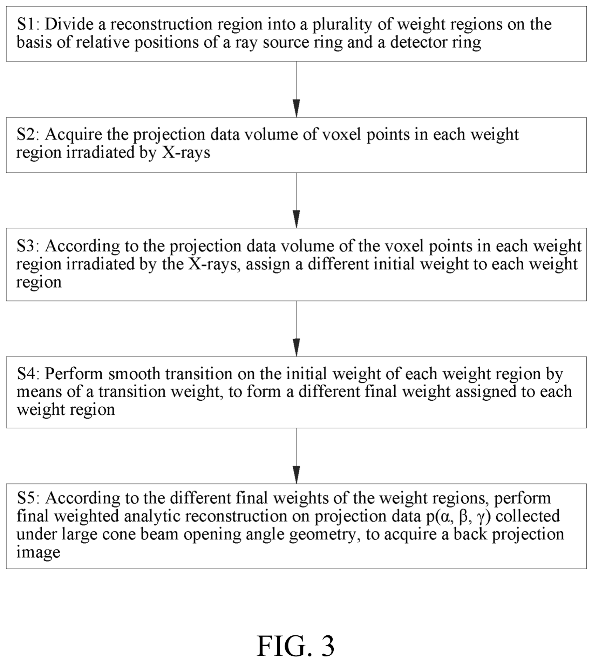

As shown in FIG. 3, the weighted analytic filtered back projection reconstruction method for asymmetric cone angle artifacts according to the embodiment of the present disclosure specifically includes steps S1 to S4:
S1: A reconstruction region is divided into a plurality of weight regions on the basis of relative positions of a ray source ring and a detector ring.

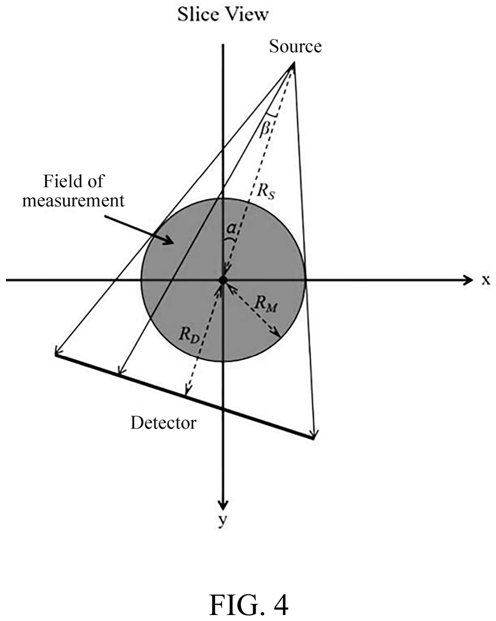

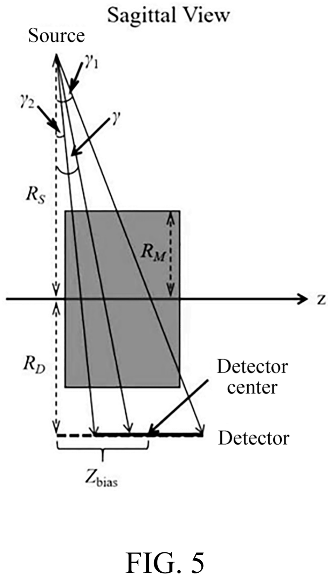

Specifically, in this embodiment, the ray source ring and the detector ring are mutually staggered to form asymmetric cone angle artifacts. Moreover, as shown in FIG. 4 and FIG. 5, a space coordinate system is constructed by using a center of the detector ring as a circle center O, using an axis of the detector ring as a Z axis, using a horizontal radial direction of the detector ring as an X axis, and using a vertical radial direction of the detector ring as a Y axis. In the space coordinate system, cone beam opening angles of the X-rays received by the detector ring fall within a range $[\gamma_2, \gamma_1]$, and a rotation angle $\varphi$ corresponding to coordinates $\mathbf{v} = (x, y, z)$ of a voxel point satisfies $x = -r\sin\varphi$ and $y = r\cos\varphi$.

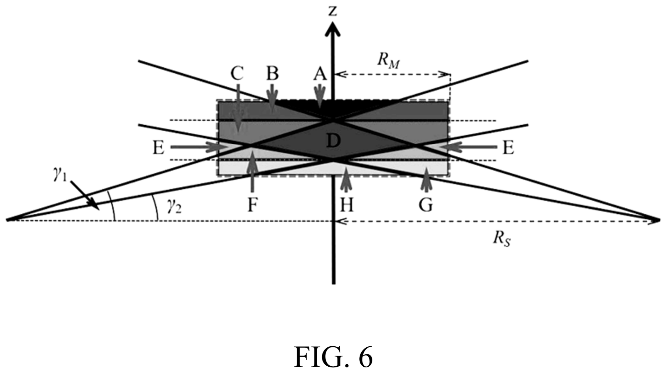

As shown in FIG. 6, in this embodiment, the reconstruction region is divided into a total of eight weight regions A to H by using the cone beam opening angles of the X-rays on two sides of the Z axis.

A range $\omega(v, \theta)$ of angles at which the eight weight regions are irradiated by the X-rays is as follows:

$$\omega(\mathbf{v},\theta)=\begin{cases}\phi \text{ (empty set)} & \text{if } r < c_1 z - R_S \text{ (Region A)} \\[6pt] \left[\varphi - \dfrac{\pi}{2} - \theta_1, \varphi + \dfrac{\pi}{2} + \theta_1\right] & \text{else if } r \geq c_1 z - R_S \geq 0 \text{ (Region B)} \\[6pt] \left[\varphi - \dfrac{\pi}{2} - \theta_1, \varphi + \dfrac{\pi}{2} + \theta_1\right] & \text{else if } c_2 z - R_S \geq r \geq R_S - c_1 z > 0 \text{ (Region C)} \\[6pt] \left[\varphi - \pi, \varphi + \pi\right] & \text{else if } r < R_S - c_1 z \text{ and } r \leq c_2 z - R_S \text{ (Region D)} \\[6pt] \left[\varphi - \dfrac{\pi}{2} - \theta_1, \varphi - \dfrac{\pi}{2} - \theta_2\right] \cup \left[\varphi + \dfrac{\pi}{2} + \theta_2, \varphi + \dfrac{\pi}{2} + \theta_1\right] & \text{else if } r \geq R_S - c_1 z > 0 \text{ and } r > c_2 z - R_S > 0 \text{ (Region E)} \\[6pt] \left[\varphi - \pi, \varphi - \dfrac{\pi}{2} - \theta_2\right] \cup \left[\varphi + \dfrac{\pi}{2} + \theta_2, \varphi + \pi\right] & \text{else if } R_S - c_1 z > r > c_2 z - R_S > 0 \text{ (Region F)} \\[6pt] \left[\varphi - \pi, \varphi - \dfrac{\pi}{2} - \theta_2\right] \cup \left[\varphi + \dfrac{\pi}{2} + \theta_2, \varphi + \pi\right] & \text{else if } r \geq c_2 z - R_S \geq 0 \text{ (Region G)} \\[6pt] \phi \text{ (empty set)} & \text{else (Region H)}\end{cases}$$

where $c_1 = \cot \gamma_1$, $c_2 = \cot \gamma_2$, and the definitions of $\theta_1$ and $\theta_2$ are as follows:

$$\theta_1 = \begin{cases} \arcsin \dfrac{R_S - c_1 z}{r} - \arctan \dfrac{\sqrt{r^2 - (R_S - c_1 z)^2}}{c_1 z} & \text{if } r > |R_S - c_1 z| \\[12pt] \operatorname{sgn}(R_S - c_1 z)\dfrac{\pi}{2} & \text{else} \end{cases}$$

$$\theta_2 = \begin{cases} \arcsin \dfrac{R_S - c_2 z}{r} - \arctan \dfrac{\sqrt{r^2 - (R_S - c_2 z)^2}}{c_2 z} & \text{if } r > |R_S - c_2 z| \\[12pt] \operatorname{sgn}(R_S - c_2 z)\dfrac{\pi}{2} & \text{else} \end{cases}$$

where $R_S$ represents a vertical distance from a ray source to a center of an FOV; z represents a Z-axis coordinate of a voxel point v; r represents the length of a vector (x, y) in an XY plane; and $\varphi$ represents an angle formed between the vector (x, y) in the XY plane and the Y axis.

S2: The projection data volume of voxel points in each weight region irradiated by the X-rays is acquired.

Specifically, in this embodiment, the projection data volume $\phi(v, \theta)$ of the voxel points in each weight region irradiated by the X-rays is calculated by the following formula:

$$\Phi(\mathbf{v},\theta) = \begin{cases} 0 & \text{if } r < c_1 z - R_S \text{ (Region A)} \\ \pi + 2\theta_1 & \text{else if } r \geq c_1 z - R_S \geq 0 \text{ (Region B)} \\ \pi + 2\theta_1 & \text{else if } c_2 z - R_S \geq r \geq R_S - c_1 z > 0 \text{ (Region C)} \\ 2\pi & \text{else if } r < R_S - c_1 z \text{ and } r \leq c_2 z - R_S \text{ (Region D)} \\ 2\theta_1 - 2\theta_2 & \text{else if } r \geq R_S - c_1 z > 0 \text{ and } r > c_2 z - R_S > 0 \text{ (Region E)} \\ \pi - 2\theta_2 & \text{else if } R_S - c_1 z > r > c_2 z - R_S > 0 \text{ (Region F)} \\ \pi - 2\theta_2 & \text{else if } r \geq R_S - c_2 z \geq 0 \text{ (Region G)} \\ 0 & \text{else (Region H)} \end{cases}$$

S3: According to the projection data volume of the voxel points in each weight region irradiated by the X-rays, a different initial weight is assigned to each weight region.

In this embodiment, according to the projection data volume of the voxel points in each weight region irradiated by the X-rays, the eight weight regions A to H are divided into four types of regions: a non-irradiated region, a partially irradiated region, a fully irradiated region, and a non-fixed irradiated region. An initial weight 0 is assigned to the non-irradiated region. An initial weight $0 < W_{PS}(v, \theta) < 1$ is assigned to the partially irradiated region and the non-fixed irradiated region. An initial weight $W_{FS}(v,\theta) = 1$ is assigned to the fully irradiated region.

Specifically, the weight region A and the weight region H are divided as non-irradiated regions; the weight region B and the weight region G are divided as regions irradiated at an angle less than 180°; the weight region C and the weight region F are divided as regions irradiated at an angle equal to or greater than 180° and less than 360°; the weight region D is divided as a 360° fully irradiated region; and the weight region E is divided as a non-fixed irradiated region. The regions irradiated at an angle less than 180° and the regions irradiated at an angle equal to or greater than 180° and less than 360° are all partially irradiated regions, but the specific initial weight $W_{PS}(v, \theta)$ assigned thereto has certain differences as follows:

If the region irradiated at an angle equal to or greater than 180° and less than 360° is the weight region C, i.e., $c_2 z - R_S > R_S - c_1 z$ is satisfied, then

$$W_{PS}(\mathbf{v},\theta) = \begin{cases} 0 & \text{if } \theta < \varphi - \pi/2 - \theta_1 \\ 1 + s(\dfrac{\theta - \varphi + \pi/2}{\theta_1}) & \text{else if } \theta < \varphi - \pi/2 + \theta_1 \\ 2 & \text{else if } \theta < \varphi + \pi/2 - \theta_1 \\ 1 - s(\dfrac{\theta - \varphi - \pi/2}{\theta_1}) & \text{else if } \theta < \varphi + \pi/2 + \theta_1 \\ 0 & \text{else} \end{cases}$$

If the region irradiated at an angle equal to or greater than 180° and less than 360° is the weight region F, i.e., $R_S - c_1 z \geq c_2 z - R_S$ is satisfied, then

$$W_{PS}(\mathbf{v},\theta) = \begin{cases} 2 & \text{if } \theta < \varphi - \pi/2 + \theta_2 \\ 1 + s(\dfrac{\theta - \varphi + \pi/2}{\theta_2}) & \text{else if } \theta < \varphi - \pi/2 - \theta_2 \\ 0 & \text{else if } \theta < \varphi + \pi/2 + \theta_2 \\ 1 - s(\dfrac{\theta - \varphi - \pi/2}{\theta_2}) & \text{else if } \theta < \varphi + \pi/2 - \theta_2 \\ 2 & \text{else} \end{cases}$$

where a function $s(t) = \sin(\pi t/2)$.

For the region irradiated at an angle less than 180° and/or the non-fixed irradiated region, i.e., the weight region B, the weight region G, and/or the weight region E,

$$W_{PS}(\mathbf{v},\theta) = \begin{cases} \min(2, \dfrac{2\pi}{\Phi(\mathbf{v},\theta)}) & \text{if } \theta \in \omega(\mathbf{v},\theta) \\ 0 & \text{else} \end{cases}$$

For the 360° fully irradiated region, i.e., the weight region D, the same weight $W_{FS}(v,\theta) = 1$ is assigned to all data.

S4: Smooth transition is performed on the initial weight of each weight region by means of a transition weight, to form a final weight assigned to each weight region.

Specifically, in this embodiment, the transition weight $W_T(v, \theta)$ is introduced to perform smooth transition on the initial weight of each weight region, to form the final weight $W_C(v,\theta) = (1 - W_T(v,\theta))W_{FS}(v,\theta) + W_T(v,\theta)W_{PS}(\mathbf{v}, \theta)$ assigned to each weight region.

For a non-360° fully irradiated region (i.e., the other seven weight regions than the weight region D), $W_T(v, \theta)=1$.

For the 360° fully irradiated region (i.e., the weight region D),

$$W_T(\mathbf{v},\theta) = \frac{1}{2} \begin{cases} 0 & \text{if } r < r_0 - \Delta r \\ 1 + s(\dfrac{r - r_0}{\Delta r}) & \text{else if } r \le r_0 + \Delta r \\ 2 & \text{else} \end{cases}$$

where $\Delta r = R_M^2/2R_S$, $r_0 = \min(R_S - c_1 z, c_2 z - R_S) - \Delta r$, and $R_M$ is the radius of the FOV (as shown in FIG. 4).

S5: According to the different final weights of the weight regions, final weighted analytic reconstruction is performed on the projection data $p(\alpha, \beta, \gamma)$ collected under large cone beam opening angle geometry, to acquire a back projection image.

Specifically, in this embodiment, final weighted analytic reconstruction is performed by the following formula:

$$f(\mathbf{v}) = \frac{1}{2} \int W_C(\mathbf{v},\theta) h(\xi) * p(\theta, \xi, \gamma) \mathrm{d}\theta$$

where $p(\theta, \xi, y)$ is the parallel beam geometric projection data obtained after weighted rearrangement of the $p(\alpha, \beta, \gamma)$, $\xi$ is a projection image horizontal index variable after the rearrangement, and $h(\xi)$ is a filter kernel.

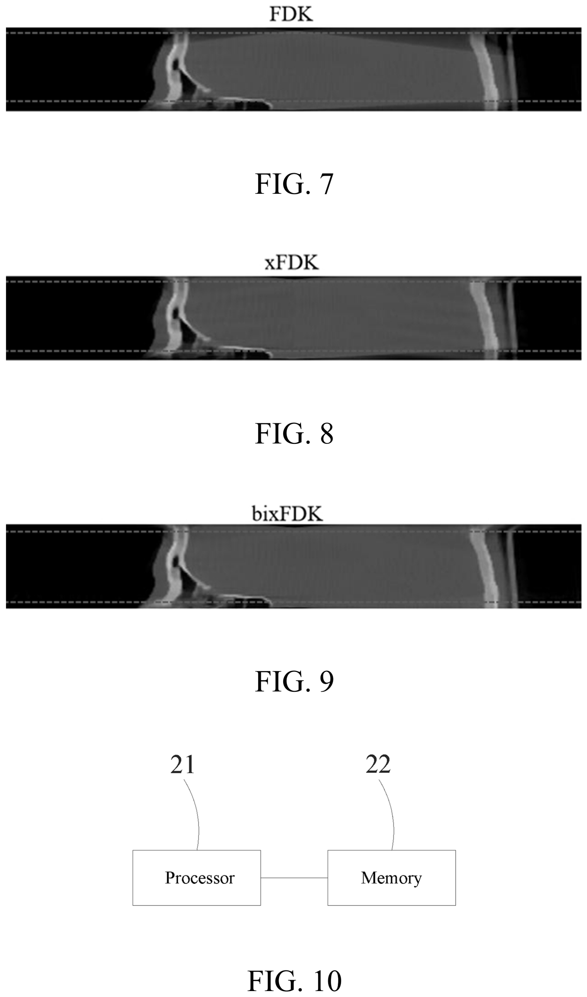

As shown in FIG. 7 to FIG. 9, FIG. 7 is a projection image reconstructed by a generic FDK algorithm, FIG. 8 is a projection image reconstructed by a generic xFDK weighting algorithm, and FIG. 9 is a projection image reconstructed by a bixFDK weighting algorithm in this embodiment. From the figures, there are obvious cone angle shadows at the upper and lower edges of the reconstruction result obtained by the generic FDK algorithm. By the xFDK algorithm, only a shadow at the upper edge can be reduced, and there is still a shadow in a region below a dashed line at a lower edge. By the bixFDK algorithm in this embodiment, shadows at the upper and lower edges are significantly reduced.

Therefore, by the steps S1 to S5, an asymmetric cone angle artifact removal method for analytic reconstruction in static CT is designed and implemented. The method can effectively estimate and compensate for asymmetric cone angle artifacts caused by staggering of ray sources and detectors. After analytic reconstruction is weighted according to the method, a reconstructable range of a single axial scan in static CT can be greatly improved, so that the uniformity and accuracy of a CT value at an axial scan analytic FBP reconstruction edge layer can be significantly improved. The utilization rate of a ray dose in static CT is indirectly improved by expanding the reconstructable range, and the ray dose required for static CT imaging is effectively reduced. In addition, it can be understood that in this embodiment, a weighting formula only applicable to a symmetric cone angle configuration system is extended to be also applicable to an asymmetric cone angle configuration system, so that the method can be applied to geometric configuration in static CT.

Based on the weighted analytic filtered back projection reconstruction method, the present disclosure further provides a back projection reconstruction system. As shown in FIG. 10, the back projection reconstruction system includes one or more processors 21 and a memory 22. The memory 22 is coupled to the processors 21 and is configured to store one or more programs. When the one or more programs are executed by the one or more processors 21, the one or more processors 21 implement the weighted analytic filtered back projection reconstruction method as described in the above embodiment.

The processors 21 are configured to control overall operations of the back projection reconstruction system, to perform all or some steps of the weighted analytic filtered back projection reconstruction method. The processors 21 may be central processing units (CPU), graphic processing units (GPU), field programmable gate arrays (FPGA), application-specific integrated circuits (ASIC), digital signal processing (DSP) chips, or the like. The memory 22 is configured to store various types of data to support operations on the back projection reconstruction system. The data may include, for example, any application program or an instruction of method for operations on the back projection reconstruction system, and data related to the application program. The memory 22 may be implemented by any type of volatile or non-volatile storage device or a combination thereof, for example, a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, or the like.

In an exemplary embodiment, the back projection reconstruction system may be specifically implemented by a computer chip or an entity, or implemented by a product having a certain function, to perform the weighted analytic filtered back projection reconstruction method, and achieve a technical effect consistent with the method. A typical embodiment is a computer. Specifically, the computer may be, for example, a personal computer, a laptop, an in-car human-machine interaction device, a cellular phone, a camera phone, a smartphone, a personal digital assistant, a media player, a navigation device, an email device, a game console, a tablet computer, a wearable device, or any combination of the devices.

In another exemplary embodiment, the present disclosure further provides a computer-readable storage medium including a program instruction. The program instruction, when executed by a processor, performs the steps of the weighted analytic filtered back projection reconstruction method in any of the above embodiments. For example, the computer-readable storage medium may be the aforementioned memory including the program instruction, and the program instruction may be executed by a processor of a back projection reconstruction system to perform the weighted analytic filtered back projection reconstruction method, and achieve a technical effect consistent with the method.

Based on the above, the weighted analytic filtered back projection reconstruction method and system for asymmetric cone angle artifacts provided in the embodiments of the present disclosure have the following beneficial effects:

1. The method and system can effectively estimate and compensate for asymmetric cone angle artifacts caused by staggering of ray sources and detectors. After analytic reconstruction is weighted according to the method, a reconstructable range of a single axial scan in static CT can be greatly improved, and the ray dose required for static CT imaging is effectively reduced.

2. A weighting formula only applicable to a symmetric cone angle configuration system is extended to be also applicable to an asymmetric cone angle configuration system, so that the method can be applied to geometric configuration in static CT.

The weighted analytic filtered back projection reconstruction method and system for asymmetric cone angle artifacts provided by the present disclosure are described in detail above. Any obvious change made by a person of ordinary skill in the art to the present disclosure without departing from the essence of the present disclosure shall constitute an infringement of the patent right of the present disclosure, and the person shall bear corresponding legal responsibility.

**Claims**

1. A weighted analytic filtered back projection reconstruction method for asymmetric cone angle artifacts, comprising the following steps:

   dividing a reconstruction region into a plurality of weight regions on the basis of relative positions of a ray source ring and a detector ring, wherein the ray source ring and the detector ring are mutually staggered to form asymmetric cone angle artifacts;
   acquiring the projection data volume of voxel points in each weight region irradiated by X-rays;
   according to the projection data volume of the voxel points in each weight region irradiated by the X-rays, assigning a different initial weight to each weight region;
   performing smooth transition on the initial weight of each weight region by means of a transition weight, to form a final weight assigned to each weight region; and
   according to the different final weights of the weight regions, performing final weighted analytic reconstruction on projection data $p(\alpha, \beta, \gamma)$ collected under large cone beam opening angle geometry, to acquire a back projection image.

2. The weighted analytic filtered back projection reconstruction method according to claim 1, wherein the dividing a reconstruction region into a plurality of weight regions on the basis of relative positions of a ray source ring and a detector ring specifically comprises:

   constructing a space coordinate system by using a center of the detector ring as a circle center O, using an axis of the detector ring as a Z axis, using a horizontal radial direction of the detector ring as an X axis, and using a vertical radial direction of the detector ring as a Y axis,
   wherein in the space coordinate system, cone beam opening angles of the X-rays received by the detector ring fall within a range $[\gamma_2, \gamma_1]$, and a rotation angle $\varphi$ corresponding to coordinates $\mathbf{v} = (x, y, z)$ of a voxel point satisfies $x = -r\sin\varphi$ and $y = r\cos\varphi$; and
   dividing the reconstruction region into a total of eight weight regions A to H by using the cone beam opening angles of the X-rays on two sides of the Z axis,
   wherein a range $\omega(v, \theta)$ of angles at which the eight weight regions are irradiated by the X-rays is as follows:

$$\omega(\mathbf{v}, \theta) = \begin{cases} \phi \text{ (empty set)} & \text{if } r < c_1 z - R_S \text{ (Region A)} \\[2mm] \left[\varphi - \dfrac{\pi}{2} - \theta_1, \varphi + \dfrac{\pi}{2} + \theta_1\right] & \text{else if } r \geq c_1 z - R_S \geq 0 \text{ (Region B)} \\[2mm] \left[\varphi - \dfrac{\pi}{2} - \theta_1, \varphi + \dfrac{\pi}{2} + \theta_1\right] & \text{else if } c_2 z - R_S \geq r \geq R_S - c_1 z > 0 \text{ (Region C)} \\[2mm] \left[\varphi - \pi, \varphi + \pi\right] & \text{else if } r < R_S - c_1 z \text{ and } r \leq c_2 z - R_S \text{ (Region D)} \\[2mm] \left[\varphi - \dfrac{\pi}{2} - \theta_1, \varphi - \dfrac{\pi}{2} - \theta_2\right] \cup \left[\varphi + \dfrac{\pi}{2} + \theta_2, \varphi + \dfrac{\pi}{2} + \theta_1\right] & \text{else if } r \geq R_S - c_1 z > 0 \text{ and } r > c_2 z - R_S > 0 \text{ (Region E)} \\[2mm] \left[\varphi - \pi, \varphi - \dfrac{\pi}{2} - \theta_2\right] \cup \left[\varphi + \dfrac{\pi}{2} + \theta_2, \varphi + \pi\right] & \text{else if } R_S - c_1 z > r > c_2 z - R_S > 0 \text{ (Region F)} \\[2mm] \left[\varphi - \pi, \varphi - \dfrac{\pi}{2} - \theta_2\right] \cup \left[\varphi + \dfrac{\pi}{2} + \theta_2, \varphi + \pi\right] & \text{else if } r \geq c_2 z - R_S \geq 0 \text{ (Region G)} \\[2mm] \phi \text{ (empty set)} & \text{else (Region H)} \end{cases}$$

   where $c_1 = \cot \gamma_1$, $c_2 = \cot \gamma_2$, and the definitions of $\theta_1$ and $\theta_2$ are as follows:

$$\theta_1 = \begin{cases} \arcsin \dfrac{R_S - c_1 z}{r} - \arctan \dfrac{\sqrt{r^2 - (R_S - c_1 z)^2}}{c_1 z} & \text{if } r > |R_S - c_1 z| \\[4mm] \operatorname{sgn}(R_S - c_1 z) \dfrac{\pi}{2} & \text{else} \end{cases}$$

$$\theta_2 = \begin{cases} \arcsin\dfrac{R_S - c_2 z}{r} - \arctan\dfrac{\sqrt{r^2 - (R_S - c_2 z)^2}}{c_2 z} & \text{if } r > |R_S - c_2 z| \\[2em] \operatorname{sgn}(R_S - c_2 z)\dfrac{\pi}{2} & \text{else} \end{cases}$$

where $R_S$ represents a vertical distance from a ray source to a center of an FOV; z represents a Z-axis coordinate of a voxel point v; r represents the length of a vector (x, y) in an XY plane; and $\varphi$ represents an angle formed between the vector (x, y) in the XY plane and the Y axis.

3. The weighted analytic filtered back projection reconstruction method according to claim 2, wherein the projection data volume $\phi(v, \theta)$ of the voxel points in each weight region irradiated by the X-rays is calculated by the following formula:

$$\Phi(\mathbf{v}, \theta) = \begin{cases} 0 & \text{if } r < c_1 z - R_S \text{ (Region A)} \\[0.5em] \pi + 2\theta_1 & \text{else if } r \geq c_1 z - R_S \geq 0 \text{ (Region B)} \\[0.5em] \pi + 2\theta_1 & \text{else if } c_2 z - R_S \geq r \geq R_S - c_1 z > 0 \text{ (Region C)} \\[0.5em] 2\pi & \text{else if } r < R_S - c_1 z \text{ and } r \leq c_2 z - R_S \text{ (Region D)} \\[0.5em] 2\theta_1 - 2\theta_2 & \text{else if } r \geq R_S - c_1 z > 0 \text{ and } r > c_2 z - R_S > 0 \text{ (Region E)} \\[0.5em] \pi - 2\theta_2 & \text{else if } R_S - c_1 z > r > c_2 z - R_S > 0 \text{ (Region F)} \\[0.5em] \pi - 2\theta_2 & \text{else if } r \geq R_S - c_2 z \geq 0 \text{ (Region G)} \\[0.5em] 0 & \text{else (Region H)} \end{cases}$$

.

4. The weighted analytic filtered back projection reconstruction method according to claim 3, wherein the assigning a different initial weight to each weight region specifically comprises:

according to the projection data volume $\phi(v, \theta)$ of the voxel points in each weight region irradiated by the X-rays, dividing the eight weight regions A to H into: a non-irradiated region, a partially irradiated region, a fully irradiated region, and a non-fixed irradiated region;
assigning an initial weight 0 to the non-irradiated region;
assigning an initial weight $0 < W_{PS}(v, \theta) < 1$ to the partially irradiated region and the non-fixed irradiated region; and
assigning an initial weight $W_{FS}(v, \theta) = 1$ to the fully irradiated region.

5. The weighted analytic filtered back projection reconstruction method according to claim 4, wherein

the weight region A and the weight region H are divided as non-irradiated regions; the weight region B and the weight region G are divided as regions irradiated at an angle less than 180°; the weight region C and the weight region F are divided as regions irradiated at an angle equal to or greater than 180° and less than 360°; the weight region D is divided as a 360° fully irradiated region; and the weight region E is divided as a non-fixed irradiated region;
if the region irradiated at an angle equal to or greater than 180° and less than 360° is the weight region C, i.e., $c_2 z - R_S > R_S - c_1 z$ is satisfied, then

$$W_{PS}(\mathbf{v},\theta) = \begin{cases} 0 & \text{if } \theta < \varphi - \pi/2 - \theta_1 \\ 1 + s(\dfrac{\theta - \varphi + \pi/2}{\theta_1}) & \text{else if } \theta < \varphi - \pi/2 + \theta_1 \\ 2 & \text{else if } \theta < \varphi + \pi/2 - \theta_1 \\ 1 - s(\dfrac{\theta - \varphi - \pi/2}{\theta_1}) & \text{else if } \theta < \varphi + \pi/2 + \theta_1 \\ 0 & \text{else} \end{cases}$$

if the region irradiated at an angle equal to or greater than 180° and less than 360° is the weight region F, i.e., $R_S - c_1 z \geq c_2 z - R_S$ is satisfied, then

$$W_{PS}(\mathbf{v},\theta) = \begin{cases} 2 & \text{if } \theta < \varphi - \pi/2 + \theta_2 \\ 1 + s(\dfrac{\theta - \varphi + \pi/2}{\theta_2}) & \text{else if } \theta < \varphi - \pi/2 - \theta_2 \\ 0 & \text{else if } \theta < \varphi + \pi/2 + \theta_2 \\ 1 - s(\dfrac{\theta - \varphi - \pi/2}{\theta_2}) & \text{else if } \theta < \varphi + \pi/2 - \theta_2 \\ 2 & \text{else} \end{cases}$$

where a function $s(t)=\sin(\pi t/2)$;
for the region irradiated at an angle less than 180° and/or the non-fixed irradiated region,

$$W_{PS}(\mathbf{v},\theta) = \begin{cases} \min(2, \dfrac{2\pi}{\Phi(\mathbf{v},\theta)}) & \text{if } \theta \in \omega(\mathbf{v},\theta) \\ 0 & \text{else} \end{cases}$$

for the 360° fully irradiated region, the same weight $W_{FS}(v, \theta) = 1$ is assigned to all data.

**6.** The weighted analytic filtered back projection reconstruction method according to claim 5, wherein the performing smooth transition on the initial weight of each weight region by means of a transition weight, to form a final weight assigned to each weight region specifically comprises:

introducing the transition weight $W_T(v, \theta)$ to perform smooth transition on the initial weight of each weight region, to form the final weight $W_C(v,\theta) = (1 - W_T(v,\theta))W_{FS}(v,\theta) + W_T(v,\theta)W_{PS}(v,\theta)$,
wherein for a non-360° fully irradiated region, $W_T(v, \theta)=1$; and
for the 360° fully irradiated region,

$$W_{\mathrm{T}}(\mathbf{v},\theta) = \frac{1}{2}\begin{cases} 0 & \text{if } r < r_0 - \Delta r \\ 1 + s(\dfrac{r - r_0}{\Delta r}) & \text{else if } r \leq r_0 + \Delta r \\ 2 & \text{else} \end{cases}$$

where $\Delta r = R_M^2/2R_S$, $r_0 = \min(R_S - c_1 z, c_2 z - R_S) - \Delta r$, and $R_M$ is the radius of the FOV.

7. The weighted analytic filtered back projection reconstruction method according to claim 6, wherein

   final weighted analytic reconstruction is performed by the following formula:

$$f(\mathbf{v}) = \frac{1}{2}\int W_{\mathrm{C}}(\mathbf{v},\theta)h(\xi) * p(\theta,\xi,\gamma)\mathrm{d}\theta$$

   where $p(\theta, \xi, \gamma)$ is the parallel beam geometric projection data obtained after weighted rearrangement of the $p(\alpha, \beta, \gamma)$, $\xi$ is a projection image horizontal index variable after the rearrangement, and $h(\xi)$ is a filter kernel.

8. A back projection reconstruction system, comprising a processor and a memory, the processor reading a computer program in the memory, and performing the following operations:

   dividing a reconstruction region into a plurality of weight regions on the basis of relative positions of a ray source ring and a detector ring, wherein the ray source ring and the detector ring are mutually staggered to form asymmetric cone angle artifacts;
   according to the projection data volume of voxel points in each weight region irradiated by the X-rays, assigning a different initial weight to each weight region;
   performing smooth transition on the initial weight of each weight region by means of a transition weight, to form a final weight assigned to each weight region; and
   according to the different final weights of the weight regions, performing final weighted analytic reconstruction on projection data $p(\alpha, \beta, \gamma)$ collected under large cone beam opening angle geometry, to acquire a back projection image.

9. The back projection reconstruction system according to claim 8, wherein
   the ray source ring is formed by a plurality of X-ray sources surrounding in a circle, the detector ring is formed by a plurality of X-ray detectors surrounding in a circle, and the detector ring is arranged on an inner side of the ray source ring.

10. A computer-readable storage medium comprising a program instruction, the program instruction, when executed by a processor, implementing the weighted analytic filtered back projection reconstruction method according to any one of claims 1 to 7.

FIG. 1

FIG. 2

S1: Divide a reconstruction region into a plurality of weight regions on the basis of relative positions of a ray source ring and a detector ring

S2: Acquire the projection data volume of voxel points in each weight region irradiated by X-rays

S3: According to the projection data volume of the voxel points in each weight region irradiated by the X-rays, assign a different initial weight to each weight region

S4: Perform smooth transition on the initial weight of each weight region by means of a transition weight, to form a different final weight assigned to each weight region

S5: According to the different final weights of the weight regions, perform final weighted analytic reconstruction on projection data $p(\alpha, \beta, \gamma)$ collected under large cone beam opening angle geometry, to acquire a back projection image

FIG. 3

FIG. 4

FIG. 5

FIG. 6

FDK

FIG. 7

xFDK

FIG. 8

bixFDK

FIG. 9

21                          22

| Processor | Memory |

FIG. 10

**EP 4 645 236 A1**

## INTERNATIONAL SEARCH REPORT

| International application No. |
|---|
| **PCT/CN2023/105441** |

| A. | CLASSIFICATION OF SUBJECT MATTER |
|---|---|
| | G06T17/00(2006.01)i |

According to International Patent Classification (IPC) or to both national classification and IPC

| B. | FIELDS SEARCHED |
|---|---|

Minimum documentation searched (classification system followed by classification symbols)

IPC: G06T

Documentation searched other than minimum documentation to the extent that such documents are included in the fields searched

Electronic data base consulted during the international search (name of data base and, where practicable, search terms used)

CNKI, CJFD, CNTXT, VCN, VEN, ENTXT, EXTXTC: 重建, 伪影, 滤波, 反投影, 分区, 区域, 划分, 权重, 权值, 加权, reconstruction, artifact, filter, back projection, divide, region, weight

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 110060316 A (CHONGQING UNIVERSITY OF POSTS AND TELECOMMUNICATIONS) 26 July 2019 (2019-07-26) description, paragraphs 9-61 | 1-10 |
| A | CN 102521853 A (SUZHOU INSTITUTE OF BIOMEDICAL ENGINEERING AND TECHNOLOGY) 27 June 2012 (2012-06-27) entire document | 1-10 |
| A | CN 105118039 A (GUANGZHOU HUARUI TECHNOLOGY CO., LTD.) 02 December 2015 (2015-12-02) entire document | 1-10 |
| A | CN 111553959 A (INFORMATION ENGINEERING UNIVERSITY OF PLA) 18 August 2020 (2020-08-18) entire document | 1-10 |
| A | US 2011091085 A1 (DENNERLEIN, Frank et al.) 21 April 2011 (2011-04-21) entire document | 1-10 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 September 2023** | **21 September 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**21**

**INTERNATIONAL SEARCH REPORT**
Information on patent family members

| International application No. |
| --- |
| **PCT/CN2023/105441** |

| Patent document cited in search report | | | Publication date (day/month/year) | Patent family member(s) | Publication date (day/month/year) |
| --- | --- | --- | --- | --- | --- |
| CN | 110060316 | A | 26 July 2019 | None | |
| CN | 102521853 | A | 27 June 2012 | None | |
| CN | 105118039 | A | 02 December 2015 | None | |
| CN | 111553959 | A | 18 August 2020 | None | |
| US | 2011091085 | A1 | 21 April 2011 | None | |

Form PCT/ISA/210 (patent family annex) (July 2022)